# EUROPEAN PATENT SPECIFICATION

(11) **EP 2 988 771 B1**
(45) Date of publication and mention of the grant of the patent: **19.12.2018**
(21) Application number: 14788015.7
(22) Date of filing: 10.04.2014
(51) Int. Cl.: A61K 38/36, A61F 13/02, A61K 38/48, A61L 15/32, A61L 15/64

(54) **FIBRINOGEN-BASED TISSUE ADHESIVE PATCHES**
GEWEBEHEFTPFLASTER AUF BASIS VON FIBRINOGEN
PATCHS ADHÉSIFS TISSULAIRES À BASE DE FIBRINOGÈNE

(30) Priority: 22.04.2013 US 201361814355 P
(43) Date of publication of application: 02.03.2016
(73) Proprietor: Sealantium Medical Ltd., 42101 Netanya (IL)
(72) Inventor: LAUB, Orgad, 69626 Tel Aviv (IL); COHN, Daniel, 93714 Jerusalem (IL); COHEN, Eran, 49421 Petah-Tikva (IL); ZAREK Matthew, Jerusalem 9356005 (IL)
(74) Representative: Lecomte & Partners
(86) International application number: PCT/IL2014/050347
(87) International publication number: WO 2014/174509

(56) References cited:
- EP-A1- 2 556 842
- WO-A1-96/40174
- WO-A2-2008/019128
- US-A1- 2007 162 121
- US-A1- 2011 071 498
- US-A1- 2012 070 485
- US-B1- 6 503 527

## Description

### FIELD OF THE INVENTION

This invention relates generally to coagulant-containing polymer films that are used as tissue sealants. In particular, it relates to a polymer film that incorporates fibrinogen and thrombin in which the fibrinogen acts to attach the polymer film to the tissue.

### BACKGROUND OF THE INVENTION

Wound dressings, tissue coatings, and tissue adhesives are examples of devices that serve to stop or prevent leakage of blood and other bodily fluids. These dressings can serve to seal open wounds, prevent infection, and so on. Many types of wound dressings and tissue adhesives known in the literature incorporate one or more coagulants such as fibrinogen.

Numerous examples are known in the literature of coagulant-containing tissue sealant compositions. U.S. Pat. No. 5631011 discloses a tissue treatment composition comprising fibrin or fibrinogen and a polymer that is biodegradable and biocompatible. The composition acts as a glue to bind tissue, e.g. a cut and sutured blood vessel. U.S. Pat. No. 6699844 discloses a fibrin-containing tissue sealant that also contains a derivative of hyaluronic acid. U.S. Pat. No. 6162241 discloses a hemostatic tissue sealant comprising a biocompatible, biodegradable hydrogel tissue sealant comprising crosslinkable groups having incorporated therein an effective amount of a hemostatic agent to stop the flow of blood from tissue in a medically acceptable period of time. U.S. Pat. No. 6056970 discloses compositions, produced by known paper-making technology, that comprise hemostatic compounds and bioabsorbable polymers.

Methods are also known in the art for preparing compositions that can release a pharmaceutically effective agent such as a hemostatic agent from a polymeric matrix. For example, U.S. Pat. No. 6194005 discloses a method in which a powdered pharmaceutically effective agent is sprayed onto a warm lipid matrix, which thereby coats the agent. U.S. Pat. No. 6579537 discloses a method for producing inter alia a fibrinogen composition using a polyalkylene glycol. The basic method comprises producing a solution of fibrinogen and fibronectin and precipitating the fibrinogen and fibronectin by adding a polyalkylene glycol and an amino acid. U.S. Pat. Appl. Pub. No. 2012/0121532 discloses a method for preparing a dry and stable hemostatic composition. A dry hemostatic agent is mixed with a dry polymeric component in proportions such that on addition of an appropriate diluent (e.g. water), a polymeric matrix (e.g. a hydrogel) into which the hemostatic agent is incorporated.

Also known in the art are non-fibrous polymer films or coatings that incorporate a hemostatic agent such as thrombin. For example, U.S. Pat. Appl. Pub. No. 2007/0059346 discloses a film containing nitroglycerin and possibly other therapeutic agents; the film is made of a water-soluble polymer that can dissolve in the mouth of a patient.

Hemostatic wound dressings that incorporate fibrinogen are also known in the art. U.S. Pat. No. 7189410 discloses a layered fibrin sealant bandage comprising a backing layer and a hemostatic component layer containing fibrinogen, the fibrinogen acting to produce a clot when the bandage is applied to a wound. A family of patents that includes inter alia U.S. Pat. No. 6054122 discloses fibrin sealant bandages that comprise an occlusive backing, an adhesive layer on the wound-facing surface of the backing, and a layer of dry hemostatic materials (fibrinogen, thrombin, and Ca²⁺ and/or Factor XIII as necessary). The dry materials adhere to, but are not incorporated into, the adhesive layer and are exposed at the time of use. U.S. Pat. Appl. Pub. No. 2006/0155235 discloses a hemostatic compression bandage that bandage comprises a flexible backing element, a powdered hemostatic substance, and a flexible film element. In this bandage, the hemostatic substance remains as a free powder. Immediately prior to use, the flexible film element is peeled away, exposing the powder, which is then placed directly on the wound. International (PCT) Pat. Appl. Pub. No. WO2006/044882 discloses a reinforced absorbable multilayered hemostatic wound dressing that comprises a first absorbable nonwoven fabric comprising aliphatic polyester polymers, copolymers, or blends thereof reinforced by a second absorbable woven or knitted fabric comprising oxidized regenerated cellulose and thrombin and fibrinogen.U.S. Pat. Appl. Pub. No. 2011/0288462 discloses a hemostatic wound dressing that comprises a super-absorbent polymer and a hemostatic agent.

In the compositions and dressings known in the literature, the fibrin sealant component serves the dual role of adhering to the tissue and as a coagulant. In hemostatic dressings known in the literature, the backing is used to support the fibrinogen, which must therefore be used in relatively large quantities. There thus remains a need for a tissue sealant or adhesive device that uses fibrin to cause the film component to adhere to the tissue but in which the film component, rather than the fibrin component, is the primary tissue sealing component.

International patent application published WO 96/40174 discloses a fibrin sealant bandage that may be supplemented with a growth factor and/or a drug, as well as methods of production and use.

European patent application published EP 2 556 842 A1 discloses a composition in the form of solid film that comprises a layer with a fibrinogen activator and a biocompatible material and another layer with fibrinogen and a biocompatible material, wherein the fibrinogen activator and the fibrinogen are not present simultaneously in one and the same layer.

US patent published US 6,503527 B1 discloses a fibrin adhesive.

US patent application published US 2007/0162121 A1 discloses a method and a means for repair and reconstruction of ruptured ligaments and tendons.

International patent application published WO 2008/019128 A2 discloses solid dressings for treated wounded tissue in mammalian patients, such as a human.

### SUMMARY OF THE INVENTION

The invention discloses a fibrinogen-based tissue adhesive patch in accordance with appended independent claim 1 and with appended dependent claims 2-5. Its use in the treatment of a leak of a fluid into or out of a body part is also disclosed in appended claims 14 and 15.

The invention also discloses a method for producing a fibrinogen-based tissue adhesive patch in accordance with appended independent method claim 6 and with appended dependent method claims 7-13.

The invention herein disclosed is designed to meet this long-felt need. It is therefore an object of this invention to disclose a fibrinogen-based tissue adhesive patch, comprising a backing made from a film made of a biocompatible polymer and a fibrinogen sealant. It is within the essence of the invention wherein said fibrinogen sealant is incorporated into said biocompatible polymer backing. In some embodiments of the invention, said fibrinogen sealant comprises fibrinogen, thrombin, and CaCl₂. In some embodiments of the invention, said fibrinogen sealant comprises fibrinogen but does not comprise thrombin. In some embodiments of the invention, said fibrinogen sealant consists essentially of fibrinogen, thrombin, and CaCl₂. In some embodiments of the invention, said fibrinogen sealant consists essentially of fibrinogen.

It is a further object of this invention to disclose such a tissue adhesive patch, wherein said tissue adhesive patch does not include any mesh or woven component.

It is a further object of this invention to disclose such a tissue adhesive patch, wherein said tissue adhesive patch does not comprise any hemostatic agent in the form of a free powder.

It is a further object of this invention to disclose such a tissue adhesive patch as defined in any of the above, wherein said biocompatible polymer is non-permeable.

It is a further object of this invention to disclose such a tissue adhesive patch as defined in any of the above, wherein said biocompatible polymer is selected from the group consisting of polyethylene glycol - polycaprolactone copolymers; polyethylene glycol - DL-lactide copolymers; and polyethylene glycol - polycaprolactone - DL-lactide copolymers.

It is a further object of this invention to disclose such a tissue adhesive patch as defined in any of the above, wherein said backing has a thickness of about 200 µm.

It is a further object of this invention to disclose such a tissue adhesive patch as defined in any of the above, wherein said patch comprises between 0.5 mg and 8 mg of fibrinogen and between 20 IU and 1000 IU of thrombin per square centimeter of film. In some embodiments of the invention, said fibrinogen sealant comprises fibrinogen, thrombin, and CaCl₂ in a ratio of 425 : 5 : 11 by weght.

It is a further object of this invention to disclose such a tissue adhesive patch as defined in any of the above, wherein said fibrinogen sealant additionally comprises at least one additive. In some preferred embodiments of the invention, said additive is selected from the group consisting of additives for extending the adhesion half-life of said film, pharmaceutically active agents, and analgesics. In some preferred embodiments of the invention, said additive is a plasmin inhibitor for extending the adhesion half-life of said film. In some preferred embodiments of the invention, said additive is a pharmaceutically active agent for targeted or controlled release.

It is a further object of this invention to disclose a method for producing a fibrinogen-based tissue adhesive patch, wherein said method comprises: casting a polymer film from a biocompatible polymer; softening said polymer film; placing a fibrinogen sealant on at least one surface of said polymer film; and pressing said polymer film until at least a portion of said fibrinogen sealant is incorporated into the surface of said polymer film. In some embodiments of the method, said step of placing a fibrinogen sealant on at least one surface of said polymer film comprises placing a fibrinogen sealant comprising fibrinogen, thrombin, and CaCl₂ on at least one surface of said polymer film. In some embodiments of the method, said step of placing a fibrinogen sealant on at least one surface of said polymer film comprises placing a fibrinogen sealant comprising fibrinogen but not comprising thrombin on at least one surface of said polymer film. In some embodiments of the method, said step of placing a fibrinogen sealant on at least one surface of said polymer film comprises placing a fibrinogen sealant consisting essentially of fibrinogen, thrombin, and CaCl₂ on at least one surface of said polymer film. In some embodiments of the method, said step of placing a fibrinogen sealant on at least one surface of said polymer film comprises placing a fibrinogen sealant consisting essentially of fibrinogen on at least one surface of said polymer film.

It is a further object of this invention to disclose such a method, wherein said biocompatible polymer is non-permeable.

It is a further object of this invention to disclose such a method, wherein said biocompatible polymer is selected from the group consisting of polyethylene glycol - polycaprolactone copolymers; polyethylene glycol - DL-lactide copolymers; and polyethylene glycol - polycaprolactone - DL-lactide copolymers.

It is a further object of this invention to disclose the method as defined in any of the above, wherein said step of casting a polymer film comprises preparing a solution of a dry polymer in an organic solvent and evaporating said organic solvent. In some preferred embodiments of the invention, said step of preparing a solution of dry polymer in organic solvent comprises preparing a 3 - 5% (w/v) solution. In some preferred embodiments of the invention, said step of preparing a solution of dry polymer in organic solvent comprises preparing a solution of dry polymer in an organic solvent selected from the group consisting of THF, chloroform, dioxane, acetone, 1-methyl-2-pyrrolidinone, DMF, and DMA. In some particularly preferred embodiments of the invention, said step of preparing a solution of dry polymer in organic solvent comprises preparing a solution of dry polymer in THF. In some embodiments of the invention, it further comprises covering said solution during at least part of the time that said step of evaporating said organic solvent is taking place.

It is a further object of this invention to disclose the method as defined in any of the above, wherein said step of casting a polymer film comprises casting a polymer film of thickness of about 200 µm.

It is a further object of this invention to disclose the method as defined in any of the above, wherein said step of casting a polymer film comprises casting said polymer film on a smooth flat surface. In some preferred embodiments of the invention, said step of placing said polymer film on a smooth flat surface comprises placing said polymer film on a surface made of a material selected from the group consisting of glass, silicone, and polytetrafluoroethylene. In some particularly preferred embodiments of the invention, said step of placing said polymer film on a smooth flat surface comprises placing said polymer film on a glass surface. In some embodiments of the invention, the method further comprises a step of removing said polymer film from said smooth flat surface following said step of pressing said polymer film. In some embodiments of the invention, it further comprises a step of placing said polymer film in a freezer following said step of pressing said polymer and prior to said step of removing said polymer film from said smooth flat surface. In some preferred embodiments of the invention, said step of placing said polymer film in a freezer comprises placing said polymer film in a freezer at a temperature of between -25 °C and -15 °C.

It is a further object of this invention to disclose the method as defined in any of the above, wherein said step of softening said polymer film comprises heating said polymer film until said polymer film softens. In some embodiments of the invention, said step of heating said polymer film until said polymer film softens comprises heating said polymer film to a temperature of between 55 °C and 60 °C. In some embodiments of the invention, said step of pressing said polymer film is followed by a step of cooling said polymer film sufficiently slowly that the film returns substantially to its original morphology.

It is a further object of this invention to disclose such a method, wherein said step of softening said polymer film comprises softening said polymer film by using residual solvent.

It is a further object of this invention to disclose the method as defined in any of the above, wherein said step of placing a fibrinogen sealant comprising fibrinogen, thrombin, and CaCl₂ on at least one surface of said polymer film comprises placing a sufficient quantity of said fibrinogen sealant on at least one surface of said polymer film sufficient to provide between 0.5 mg and 8 mg of fibrinogen and between 20 IU and 1000 IU of thrombin per square centimeter of film.

It is a further object of this invention to disclose the method as defined in any of the above, wherein said step of placing a fibrinogen sealant comprising fibrinogen, thrombin, and CaCl₂ on at least one surface of said polymer film comprises placing a fibrinogen sealant comprising fibrinogen, thrombin, CaCl₂ and at least one additive on at least one surface of said polymer film. In some embodiments of the invention, said step of placing a fibrinogen sealant comprising fibrinogen, thrombin, CaCl₂ and at least one additive on at least one surface of said polymer film comprises placing a fibrinogen sealant comprising fibrinogen, thrombin, CaCl₂ and at least one additive selected from the group consisting of additives for extending the adhesion half-life of said polymer film, pharmaceutically active agents, and analgesics on at least one surface of said polymer film. In some preferred embodiments of the invention, said step of placing a fibrinogen sealant comprising fibrinogen, thrombin, CaCl₂ and at least one additive on at least one surface of said polymer film comprises placing a fibrinogen sealant comprising fibrinogen, thrombin, CaCl₂ and a plasmin inhibitor on at least one surface of said polymer film. In some preferred embodiments of the invention, said step of placing a fibrinogen sealant comprising fibrinogen, thrombin, CaCl₂ and at least one additive on at least one surface of said polymer film comprises placing a fibrinogen sealant comprising fibrinogen, thrombin, CaCl₂ and at least one pharmaceutically active agent for targeted or sustained release on at least one surface of said polymer film.

It is a further object of this invention to disclose the method as defined in any of the above, further comprising providing a fibrinogen sealant comprising fibrinogen, thrombin, and CaCl₂ in the form of a powder. In some embodiments of the invention, said step of placing a fibrinogen sealant comprising fibrinogen, thrombin, and CaCl₂ on at least one surface of said polymer film comprises placing said powder on said at least one surface by a method selected from the group consisting of sprinkling, spreading, spraying, and spraying a suspension of said powder in an organic solvent.

It is a further object of this invention to disclose the method as defined in any of the above, further comprising a step of placing a smooth material having a flat surface on top of said film prior to said step of pressing said polymer film.

It is a further object of this invention to disclose the method as defined in any of the above, wherein said step of pressing said polymer film comprises pressing said polymer film according to a programmed compression procedure. In some embodiments of the invention, said step of pressing said polymer film according to a programmed compression procedure comprises pressing said polymer film with a force that continuously increases to a maximum of about 50 N.

It is a further object of this invention to disclose the method as defined in any of the above, further comprising a step of removing excess fibrinogen sealant from said polymer film following said step of pressing said polymer film.

It is a further object of this invention to disclose a tissue adhesive patch as defined in any of the above, produced by a method as defined in any of the above.

It is a further object of this invention to disclose a method of treating a leak of fluid into or out of a body part, comprising applying a tissue adhesive patch as defined in any of the above in which said fibrinogen-based sealant comprises or consists essentially of fibrinogen, thrombin, and CaCl₂ to said body part, thereby causing said tissue adhesive patch to adhere to said affected artery or organ, thereby sealing said artery or organ. In some embodiments of the method of treating a leak of fluid into or out of a body part, said body part is selected from the group consisting of arteries and organs. In some embodiments, said step of applying a tissue adhesive patch comprises manually pressing said patch on the surface of said body part.

It is a further object of this invention to disclose a method of treating a leak of fluid into or out of a body part, comprising applying thrombin to said body part and applying a tissue adhesive patch as defined in any of the above in which said fibrinogen-based sealant comprises or consists essentially of fibrinogen to said body part, thereby causing said tissue adhesive patch to adhere to said affected artery or organ, thereby sealing said artery or organ. In some embodiments of the method of treating a leak of fluid into or out of a body part, said body part is selected from the group consisting of arteries and organs. In some embodiments, said step of applying a tissue adhesive patch comprises manually pressing said patch on the surface of said body part.

It is a further object of this invention to disclose such a method of treating a leak of fluid into or out of a body part as defined in any of the above, wherein said leak of fluid is selected from the group consisting of arterial bleeding; organ tissue bleeding; bile anastomosis; cerebrospinal fluid leak; dura leak; and air leak in damaged lung tissue.

It is a further object of this invention to disclose the use of a tissue adhesive patch as defined in any of the above in the treatment of a leak of fluid into or out of a body part.

It is a further object of this invention to disclose such a use, wherein said leak of fluid is selected from the group consisting of arterial bleeding; organ tissue bleeding; bile anastomosis; cerebrospinal fluid leak; dura leak; and air leak in damaged lung tissue.

### BRIEF DESCRIPTION OF THE DRAWINGS

The invention will now be described with reference to the drawings, wherein:
FIG. **1** presents a graph showing the tensile modulus of wet and dry films made from a polyethylene glycol - polycaprolactone copolymer (PECA) as function of the ratio of ethylene oxide (EO) to caprolactone (CL) units in the polymer;
FIG. **2** presents graphs showing the tensile modulus of wet polyethylene - caprolactone - lactide (PECALA) films of varying compositions in comparison to a PECA film with an EO/CL ratio of 2.0;
FIG. **3** shows DSC traces for wet and dry PECA films of different ethylene oxide / caprolactone ratios and for wet PECALA films of varying compositions;
FIG. **4** presents results of measurements of water uptake by dry PECA films of different ethylene oxide / caprolactone ratios and for dry PECALA films of varying compositions;
FIG. **5** shows a photograph of a fibrinogen-based tissue adhesive film according to one embodiment of the present invention;
FIG. **6** shows a photograph of fibrinogen-based tissue adhesive film according to one embodiment of the present invention attached to a piece of raw meat after 60 minutes of washing under running water;
FIG. **7** shows a photograph of a typical measurement of the force required to detach the adhesive film of the present invention from raw meat used as a substrate;
FIG. **8** shows photographs illustrating an in-vivo biodegradability assessment and adhesion/detachment force measurement;
FIG. **9** shows the use of an adhesive patch of the present invention to seal a 3 mm hole in a rat caecum; and,
FIG. **10** shows the use of an adhesive patch of the present invention to seal a 5 mm puncture hole in a rat liver.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of said claims.

In the following description, the terms "PEG" and "PEO" refer to polyethylene glycol and polyethylene oxide, respectively, and are used interchangeably.

In the following description, the term "PECA" refers to a PEG - polycaprolactone (PCL) block copolymer. When the term PECA is followed by a number, the number indicates the ratio of ethylene oxide repeat units in the PEG segment to caprolactone repeat units in the PCL segment. The greater the number, the more hydrophilic the PECA copolymer will be.

In the following description, the term "PELA" refers to a block copolymer of PEG with lactide, the cyclic diester of lactic acid.

In the following description, the term "PECALA" refers to a triblock PEG - PCL - lactide triblock copolymer. When the term PECALA is followed by two numbers, the first indicates the ratio of hydrophilic (PEG) to hydrophobic (PCL and lactide) repeat units, and the second to the number of lactide units per triblock flank.

In the following description, the term "about," when applied to numerical quantities, refers to a range of ±25% of the nominal value.

In preferred embodiments, the backing of the fibrinogen-based tissue adhesive of the present invention comprises a film made from a biocompatible polymer into the surface of which a fibrin-based sealant is incorporated. In more preferred embodiments, the biocompatible polymer film is made from a non-permeable material. In the most preferred embodiments, the biocompatible polymer film is made from PECA, PELA, or PECALA.

In its most basic formulation, the sealant consists essentially of fibrinogen, thrombin, and CaCl₂. In other embodiments, the sealant comprises fibrinogen, thrombin, and CaCl₂, and may contain one or more additional components. Non-limiting examples of these additional components include plasmin inhibitors, which serve to extend the adhesion half-life; pharmaceutically active agents; and analgesics.

In some embodiments, the sealant contains fibrinogen, thrombin, and CaCl₂ in a ratio of 425 : 5 : 11 by weight. This ratio corresponds to ∼200 mg pure fibrinogen, ∼250 IU thrombin, and 11 mg CaCl₂ per 441 mg of the mixture. In other embodiments of the invention, no CaCl₂ is added to the adhesive, the amount of CaCl₂ present in the thrombin as received from commercial suppliers being sufficient to act as a cofactor for the enzymatic activity of the thrombin. In preferred embodiments of the invention, the sealant is a microparticulate powder, and the amount of sealant incorporated into the film is sufficient to provide a concentration of 0.5 - 8 mg of fibrin and 20 - 10000 IU of thrombin per square centimeter of film. This concentration corresponds to about 3 - 6 mg of the 425 : 5 : 11 mixture described above. In more preferred embodiments, the concentration of fibrinogen in the adhesive is between 0.5 and 6 mg per square centimeter of film. In still more preferred embodiments, the adhesive provides about 4 mg fibrinogen and about 2 - 5 IU of thrombin per square centimeter of film. In the most preferred embodiments, the concentration of fibrinogen in the adhesive is less than 2 mg per square centimeter of film.

In some embodiments of the invention, the patch is provided as a two-component system. In these embodiments, the sealant does not contain any thrombin. In some of these embodiments, the sealant consists essentially of fibrinogen; in others, the sealant comprises fibrinogen, but may contain other components, non-limiting examples of which include plasmin inhibitors, which serve to extend the adhesion half-life; pharmaceutically active agents; and analgesics. In the embodiments in which the patch is provided as a two-component system, thrombin is provided separately; as a non-limiting example, it can be provided in a solution. In these embodiments of the invention, the thrombin component is applied to the affected body part, e.g. by spraying, and the patch containing the fibrinogen-based sealant is then applied to the affected body part. The fibrinogen component of the sealant and the thrombin applied to the affected body part then react to form fibrin, which binds the patch to the affected body part.

It is emphasized that in contrast to hemostatic patches and dressings known in the art, preferred embodiments of the tissue adhesive of the present invention do not include a mesh or woven component; the polymer film, not the fibrinogen sealant, acts to seal the tissue, while the fibrinogen acts to attach the polymer film to the tissue. Nor do preferred embodiments of the present invention comprise woven or non-woven fabrics or materials made by techniques known in paper-making technology. The present invention discloses in its preferred embodiments a tissue adhesive that comprises a single layer of polymer film into which fibrinogen and thrombin are incorporated, in contrast to multilayer hemostatic dressings known in the art (although embodiments in which additional layers are added for ease of handling or storage are not excluded from the scope of the present invention). Furthermore, in the present invention, the fibrinogen sealant component is physically incorporated into the polymer film to form a single integrated unit (as described below, in preferred embodiments, it is pressed into the surface of the film), in contrast to those hemostatic patches and dressings known in the art in which the coagulant is present as a free powder.

The incorporation of a fibrinogen sealant material into a non-permeable polymer film backing, a configuration previously unknown in the art, enables the tissue adhesive of the present invention to be used in a variety of unique applications. Non-limiting examples of applications in which the present invention can be used include covering traumatic and chronic wounds, stopping of arterial bleeding, stopping organ tissue bleeding, and sealing of other body fluids, for example, in treatment of bile anastomosis, cerebrospinal fluid and dura leaks, etc.

One important consideration for design of the patch is the physical properties of the polymer used to produce it. Some of the relationships between the composition of the polymer and its properties are given here as non-limiting examples of the types of polymer films that can be produced for use in the invention disclosed herein and thereby tailored to specific uses.

Table 1 summarizes some of the properties of PECA copolymers, as determined by GPC and ¹H-NMR, as a function of the EO/CL ratio. The molecular weights reported in the table are true molecular weights and not polystyrene equivalents.

**TABLE 1**

| EO/CL | # of CL units per side | Molecular weight of CL flank [g/mol] | Number-average MW [g/mol] | Weight-average MW [g/mol] | Polydispersity | Degree of polymerization |
|---|---|---|---|---|---|---|
| 2.0 | 34.1 | 3,890 | 16,431 | 23,660 | 1.44 | 1.72 |
| 2.4 | 28.4 | 3,240 | 18,289 | 24,873 | 1.36 | 2.00 |
| 2.8 | 24.4 | 2,780 | 24,296 | 31,585 | 1.30 | 2.73 |
| 3.2 | 21.3 | 2,430 | 19,729 | 27,621 | 1.40 | 2.54 |
| 3.6 | 18.9 | 2,160 | 21,772 | 29,174 | 1.34 | 2.83 |
| 4.0 | 17.0 | 1,940 | 16,724 | 24,585 | 1.47 | 3.02 |
| 4.4 | 15.5 | 1,770 | 20,498 | 27,058 | 1.32 | 2.84 |
| 4.8 | 14.2 | 1,620 | 19,082 | 26,334 | 1.38 | 2.85 |
| 5.2 | 13.1 | 1,490 | 19,643 | 26,124 | 1.33 | 2.89 |

One property of PECA that makes it less preferred for some applications as a material for the backing of a hemostatic patch is its relatively slow rate of biodegradation; e.g., an *in vitro* test of a number of PECA films of varying compositions showed little degradation even after three months. Therefore, in some preferred embodiments of the invention in which rapid biodegradation is desired, PECALA films, which incorporate DL-lactide units into the CL segments, and hence biodegrade much more rapidly than PECA films, are used. Table 2 summarizes the properties, as determined by GPC and ¹H-NMR spectroscopy, of PECALA films of various compositions.

**TABLE 2**

| Hydrophilic/ Hydrophobic Ratio | # of lactide units per side | Number-average MW [g/mol] | Weight-average MW [g/mol] | Polydispersity | Degree of polymerization |
|---|---|---|---|---|---|
| 2.0 | 0.34 | 20,375 | 28,117 | 1.38 | 2.02 |
| 2.0 | 0.66 | 18,295 | 24,515 | 1.34 | 2.05 |
| 2.2 | 2.81 | 21,411 | 27,834 | 1.30 | 2.24 |
| 2.2 | 3.55 | 17,254 | 25,363 | 1.47 | 1.93 |

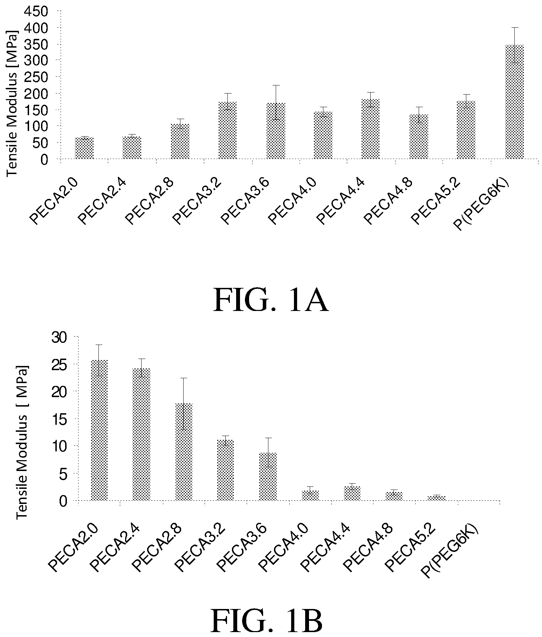

The mechanical properties of the polymer used to produce the film depend on the composition of the triblock and the molecular weight of the chain extended polymer, including the urethane linkages. Reference is now made to FIG. **1****,** which shows the tensile moduli of dry (FIG. **1A**) and wet (FIG. **1B**) PECA films as a function of the EO/CL ratio. The differences in the behavior of the dry and wet polymer films are primarily due to the ratio of the amount of hydrophilic PEG to hydrophobic PCL in the polymer films. Note that for the dry polymer films, the tensile modulus rises from ∼60 MPa for PECA2.0 to ∼160 MPa for PECA5.2, but appears to plateau at higher EO/CL ratios. In contrast, the tensile modulus of the wet polymer decreases with increasing EO/CL.

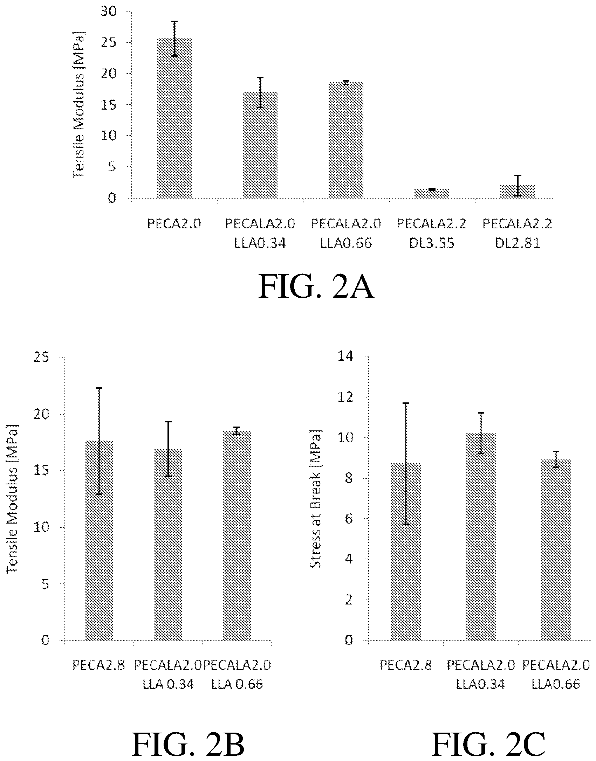

Reference is now made to FIG. **2A****,** which shows graphically the tensile moduli of several wet PECALA films. The tensile modulus of a wet PECA2.0 film is shown for comparison. Reference is now made to FIGs. **2B** and **2C****,** which show the tensile moduli and stress at break, respectively, for two different PECALA films in comparison to PECA2.8. These graphs show that PECALA's physical properties are similar to those of PECA.

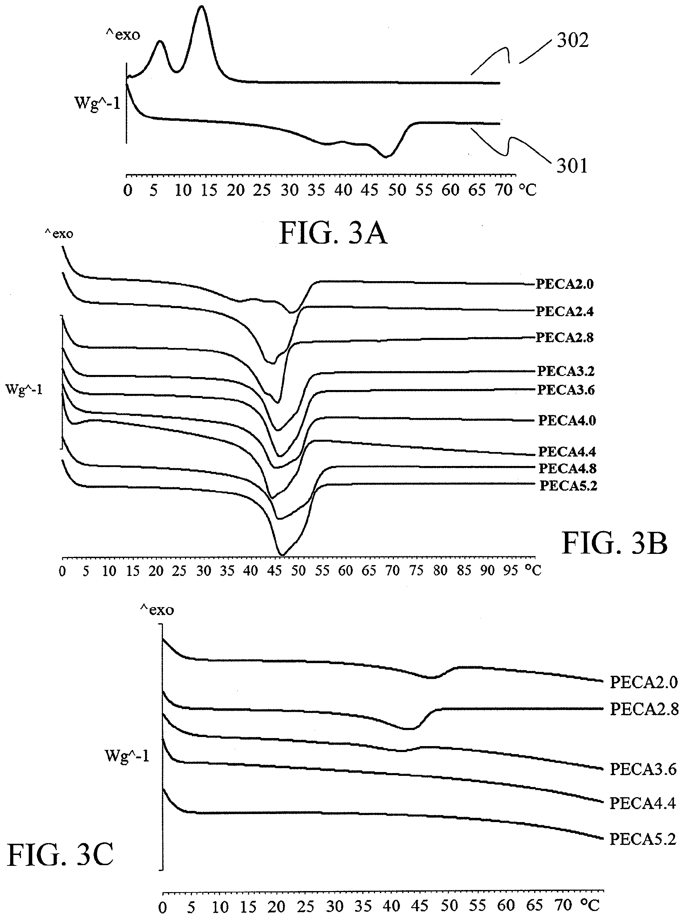

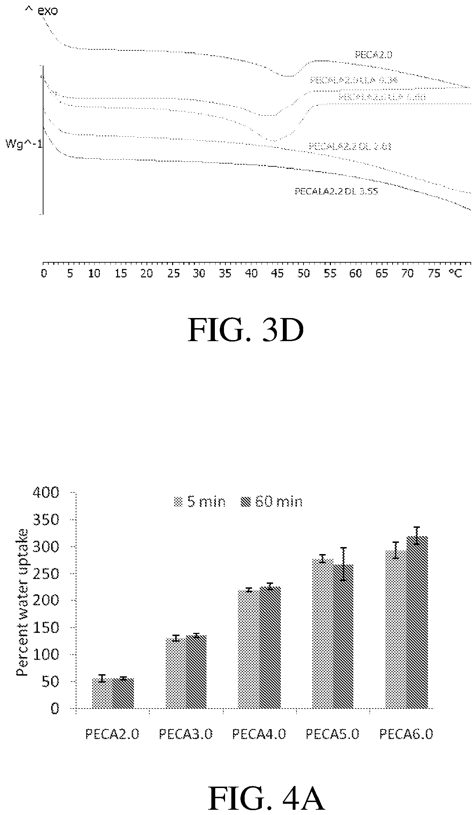

Reference is now made to FIG. **3****,** which presents DSC traces showing the thermal transitions of polymer films of various compositions. FIG. **3A** shows a trace for FECA2.0, the melt of which (trace **301** in the figure) shows evidence for two components, and in which the cooling of the melt (trace **302** in the figure) shows two distinct recrystallizations. The larger peak belongs to the caprolactone segment. FIG. **3B** shows DSC traces for dry PECA films of various EO/CL ratios, and FIG. **3C** shows DSC traces for wet PECA films of various EO/CL ratios.

Reference is now made to FIG. **3D****,** which shows DSC traces for PECALA films of various compositions, with a DSC trace of a PECA2.0 film shown for comparison. These DSC traces support the conjecture that the lactide unit disrupts the crystallinity of the caprolactone segments. The traces show that for PECALA2.2 DL 2.81 and 3.55 films, the incorporation of lactide units destroys the polymer's crystallinity.

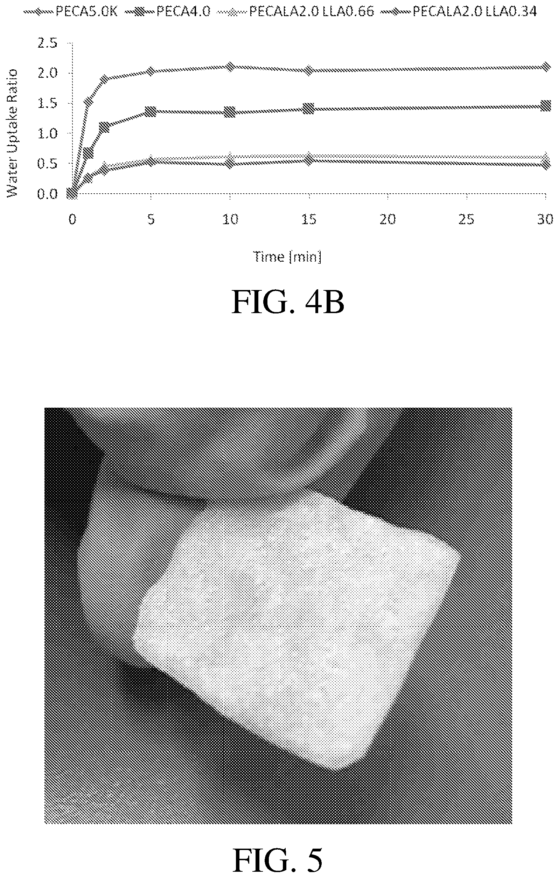

Another important physical property of the polymer films with respect to their use as backings for tissue adhesives is their water uptake. Reference is now made to FIG. **4A****,** which shows the water uptake (w/w, measured gravimetrically) after 5 and 60 minutes of a number of PECA films of various EO/CL ratios. The results show that water rapidly saturates the material. FIG. **4B** shows the water uptake (w/w, measured gravimetrically) for two PECALA compositions in comparison to two PECA compositions. As with PECA, PECALA saturates rapidly.

A preferred method of preparing the fibrinogen-based tissue adhesive of the present invention is now disclosed. A non-permeable biocompatible polymer film is cast; in preferred embodiments, the film is made from PECA, PELA, or PECALA. The film can be prepared by any method known in the art. In some preferred embodiments, a solution (typically 3 - 5%) of dry polymer in an organic solvent is prepared, and the solvent then allowed to evaporate. In preferred embodiments of the invention, the solvent is THF, but any sufficiently volatile organic solvent may be used instead. Non-limiting examples of suitable solvents include chloroform, dioxane, acetone, 1-methyl-2-pyrrolidinone, DMF, and DMA. In some preferred embodiments, the solution is covered, e.g. by perforated aluminum foil, so that the solvent does not evaporate too quickly and to prevent dust contamination, which can lead to surface defects.

The film can be of any thickness suitable for the desired final application; in typical embodiments, the film has a thickness of approximately 200 µm. The polymer film is then placed on a supporting horizontal surface made of a smooth flat material from which it will be possible to remove the film without damaging it; non-limiting examples of such surfaces include glass and sheets made from inert polymers such as silicone or polytetrafluoroethylene. The film is then softened, in preferred embodiments by heating (typically to about 55 - 60°C) or by residual solvent. In preferred embodiments in which the smooth surface is a flexible polymer sheet, a sheet of rigid material such as glass is placed between the polymer sheet and the heating element for ease of handling. The softened film is then covered by a homogeneous fibrin sealant mixture. The mixture typically comprises fibrinogen, thrombin, CaCl₂, and optionally additives, as described above. In embodiments of the invention in which the patch is provided as a two-component system, thrombin is not incorporated directly into the polymer, and the fibrin sealant comprises fibrinogen and optionally additives. Non-limiting examples of additives that can be incorporated into the mixture include additives for extending the adhesion half-life such as plasmin inhibitors, pharmaceutically active agents for targeted or sustained release, and analgesics.

The fibrin sealant mixture is then added as a powder to the softened polymer film. The powder may be added by any method known in the art. Non-limiting methods include sprinkling over the polymer film, spraying, spraying a suspension of the powder in a volatile organic solvent onto the film, or simply spreading the powder over the surface of the film. In preferred embodiments, the mixture is added in an amount sufficient to provide 0.5 - 8 mg of fibrinogen and 20 - 1000 IU of thrombin/cm² of film. In embodiments in which the patch is provided as a two-component system, as mentioned above, thrombin is not included in the fibrin sealant, but is applied separately to the affected body part, in preferred embodiments, in concentrations sufficient to provide 20 - 1000 IU / cm of film applied to the body part. The polymer film is then covered with a smooth material having a flat surface; suitable materials described above for the supporting horizontal surface are also suitable for use as the covering surface. In preferred embodiments in which the covering surface is a flexible polymer, the flexible polymer is covered with a rigid smooth material such as a glass plate in order that when the softened film is pressed (see the following paragraph), the pressure on the film is homogeneously applied.

The fibrin sealant mixture is then pressed into the surface of the softened polymer film. The pressing may be done by any method known in the art. In preferred embodiments of the invention, a programmed compression procedure is used in which the compressive force increases during the compression up to a maximum of 50 N. The actual compressive force can be adjusted according to the thickness and composition of the specific film being used; the force need only be sufficient to incorporate the powder into the surface of the softened polymer film.

After the compression, the film (still between the two smooth flat surfaces) is removed from the heating apparatus and allowed to cool to room temperature at a rate sufficiently slow such that it returns essentially to its original morphology, thus substantially retaining the mechanical, physical, and chemical properties of the film as originally formed. Once the film has returned to room temperature, it may optionally be placed in a freezer (typical freezer temperatures are -15 to -25 °C) for ∼15 minutes in order to make it easier to remove the film from the surfaces between which it sits. In embodiments in which the horizontal supporting surface and upper covering surface are made of a flexible material, freezing is generally not necessary, since the film can be peeled from the backing surfaces without risk of damage.

Excess powder, if any, is removed (e.g. by shaking or gently blowing) from the film and the film removed from the flat surface on which it was prepared. The resulting patch is ready for use and can be applied directly to tissue. The films are stable to long-term storage in a dry environment at temperatures of 2 - 25 °C. Long-term storage is typically performed by placing the film in a sealed plastic envelope in a dry refrigerated environment, typically at a temperature of 4 - 8 °C.

The invention herein disclosed is now illustrated by the following non-limiting examples that are provided to aid one of ordinary skill in the art to make and use the invention as claimed.

### EXAMPLE 1

A 4.3% w/v solution of dry PECALA in THF was prepared and poured into a glass Petri dish. The dish was covered with perforated aluminum foil and left overnight at room temperature in a fume hood. The resulting film had a thickness of approximately 200 µm.

2 cm squares of the film were excised and placed on a glass slide. The samples were then placed on a 58 °C hotplate until the polymer was softened. A powdered fibrin sealant mixture containing fibrinogen, thrombin, and CaCl₂ was sprinkled onto the surface of the softened polymer film, and the film covered with a second glass slide. The fibrin sealant mixture was then pressed into the surface of the softened polymer film by using an Instron Universal Testing Machine programmed to apply an increasing compressive force to a maximum of 50 N. Following the compression, the glass slide was slowly cooled to room temperature. The slide was then placed in a freezer at -22 °C for 15 minutes in order to help loosen the film from the slide. Excess powder was removed from the surface by shaking, and the patch removed from the glass slide using a surgical scalpel. Reference is made to FIG. **5****,** which shows a photograph of the patch thus obtained, which is ready for use.

### EXAMPLE 2

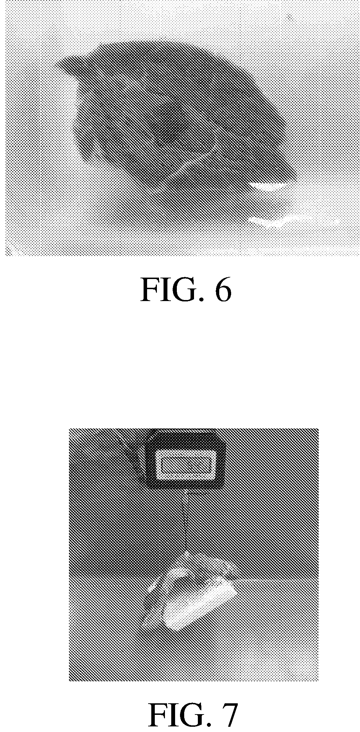

An adhesive tissue patch according to one embodiment of the present invention was prepared and attached to a piece of raw meat and then washed extensively under a stream of water. As shown in FIG. **6****,** the patch remained firmly attached even after 60 minutes of washing.

### EXAMPLE 3

Measurements were made of the adherence of an adhesive tissue patch disclosed in the present invention. A patch was prepared and applied to a piece of raw meat by manually pressing for 2 minutes. A tension and compression force gauge was used to measure the force needed to detach a 2cm × 2cm patch. FIG. **7** shows a photograph of a typical force measurement. It was found that a force of 5-7 Newton is required to detach the film.

### EXAMPLE 4

In-vivo biodegradability assessments were made following intraperitoneal (IP) implantation of adhesive patches of the present invention onto liver and intestinal (cecum) surfaces of rats. The implantations had no visible effect on the animals, which appeared healthy and gained weight during the fourteen days following the implantation.

Fourteen days after implantation of the patches, the animal was sacrificed and a gross necropsy performed, during which the abdominal cavity was opened and a macroscopic assessment of the status of the patch made. The patches could be detected attached to the implantation targets.

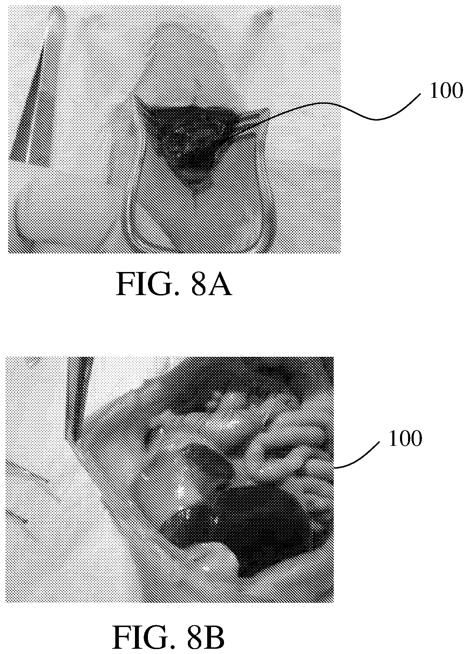

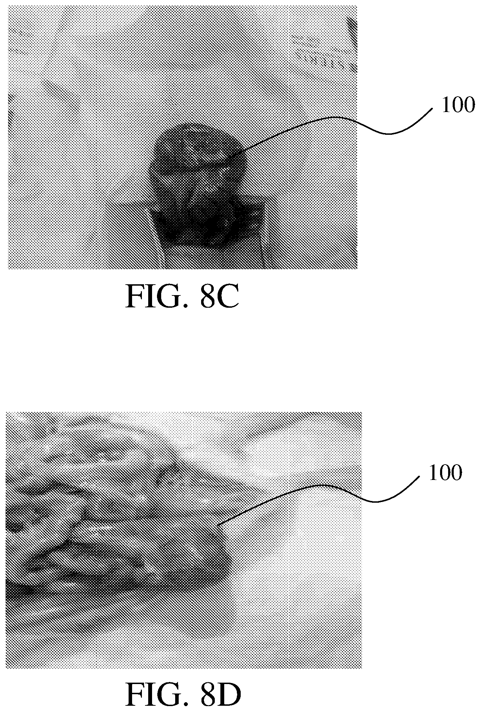

FIG. **8A** shows the implantation site for implantation of adhesive patch **100** on the liver at day 0, and FIG. **8B** shows the implantation site for implantation of adhesive patch **100** on the liver on day 14 following the implantation. FIGs. **8C** and **8D** show the implantation site for implantation on the caecum at days 0 and 14, respectively.

As can be seen from the figures, the adhesive patches remained visibly attached to the implantation site 14 days after implantation.

### EXAMPLE 5

A patch was prepared as described in Example 1 above, except that instead of a sealant mixture containing fibrinogen, thrombin, and CaCl₂, only powdered fibrinogen was sprinkled onto the surface of the softened polymer film. A solution of thrombin was sprayed onto a piece of raw meat and the patch pressed onto the meat for 2 minutes. The adherence of the patch prepared in this manner was identical to that of that of a patch applied as described in Example 3.

### EXAMPLE 6

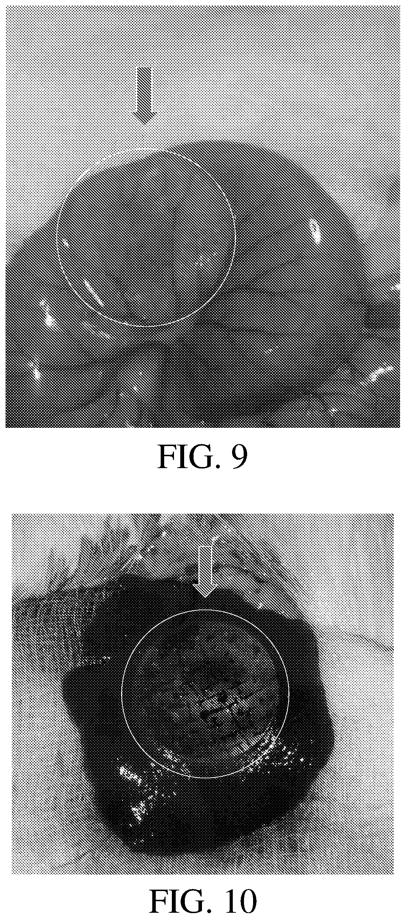

An *in vivo* study was performed to demonstrate the efficacy of the patch of the present invention in sealing damaged tissue. Rats (*n* = 6) were used as the test animals. A 2 - 3 mm hole was made in the caecum of each of tested animal using a biopsy punch. In the experimental group (*n* = 3), he hole was then covered with a 1.4 cm diameter patch of the present invention made of PECALA and containing 2 mg/cm² fibrin sealant. Reference is now made to FIG. **9****,** which shows the area of the caecum that had been punctured and then covered with the patch of the present invention (circle and arrow indicating the region). The caecum of animals in the control group (*n* = 3) was punctured, but no further treatment was performed. After the puncture was made (and sealed with the patch in the case of the test group), the caecum was returned to the abdominal cavity. The animals were followed for two weeks following the operation and then sacrificed.

All of the animals in the experimental group gained weight and showed no side effects. Necropsy data showed that the caecum of the treated animals had healed completely, the patch was absorbed into the tissue, and no local reaction could be detected. In contrast, two of the three control (untreated) animals died after experiencing severe inflammation of the abdomen.

The results of this study demonstrate that the patch of the present invention is effective in sealing intestinal leakage in a rat caecum model.

### EXAMPLE 7

A second *in vivo* study was performed to demonstrate the efficacy of the patch of the present invention in stopping severe bleeding. Rats (*n* = 6) were used as the test animals. In this study, a 6 mm hole was made in the left lobe of the liver of the test animals using a biopsy punch, resulting in severe bleeding. The hole was then covered with a 1.4 cm diameter patch of the present invention made from PECALA and containing 2 mg/cm² fibrin sealant. The device adhered well to the tissue, sealing the hole and stopping the bleeding instantly. Reference is now made to FIG. **10****,** which shows the liver of an experimental animal after the puncture and application of the patch (the puncture hole can be seen beneath the transparent patch). After hemostasis was evident, the liver was returned to the abdominal cavity. The animals were kept alive for two weeks following the treatment and then sacrificed.

All of the experimental animals gained weight and showed normal behavior with no side effects. Necropsy data showed that the liver completely recovered and that the patch had been degraded and absorbed with no reaction at the site of the injury.

These results demonstrate that the patch of the current invention is effective for stopping severe bleeding with no side effects.

## Claims

1. A fibrinogen-based tissue adhesive patch, comprising:
a backing made from a film made of a biocompatible polymer; and,
a fibrinogen sealant;
**characterized in that**
said biocompatible polymer is a polyethylene glycol - polycaprolactone - DL-lactide copolymer;
said fibrinogen sealant is incorporated into said biocompatible polymer backing; and said tissue adhesive patch does not include any mesh or woven component.

2. The tissue adhesive patch according to claim **1, characterized in that** said fibrinogen sealant consists essentially of fibrinogen, thrombin, and CaCl₂.

3. The tissue adhesive patch according to claim **1, characterized in that** said tissue adhesive patch does not comprise any hemostatic agent in the form of a free powder.

4. The tissue adhesive patch according to claim **1, characterized in that** said patch comprises between 0.5 mg and 8 mg of fibrinogen and between 20 IU and 1000 IU of thrombin per square centimeter of film.

5. The tissue adhesive patch according to claim **1, characterized in that** said fibrinogen sealant additionally comprises at least one additive.

6. A method for producing a fibrinogen-based tissue adhesive patch, **characterized in that** said method comprises:
casting a polymer film from a biocompatible polymer;
softening said polymer film;
placing a fibrinogen sealant on at least one surface of said polymer film; and,
pressing said polymer film until at least a portion of said fibrinogen sealant is incorporated into the surface of said polymer film.

7. The method according to claim **6, characterized in that** said step of placing a fibrinogen sealant on at least one surface of said polymer film comprises placing a fibrinogen sealant comprising fibrinogen, thrombin, and CaCl₂ on at least one surface of said polymer film.

8. The method according to claim **6, characterized in that** said biocompatible polymer is non-permeable.

9. The method according to claim **6, characterized in that** said biocompatible polymer is selected from the group consisting of polyethylene glycol - polycaprolactone copolymers; polyethylene glycol - DL-lactide copolymers; and polyethylene glycol - polycaprolactone - DL-lactide copolymers.

10. The method according to claim **6, characterized in that** said step of casting a polymer film comprises:
preparing a solution of a dry polymer in an organic solvent; and,
evaporating said organic solvent.

11. The method according to claim **6, characterized in that** said step of casting a polymer film comprises casting said polymer film on a smooth flat surface made of material selected from group consisting of glass, silicone, and polytetrafluoroethylene.

12. The method according to claim **11, characterized in that** said method comprises placing said polymer film in a freezer following said step of pressing said polymer and removing said polymer film from said smooth flat surface following said step of placing said polymer film in a freezer.

13. The method according to claim **6, characterized in that** said step of placing a fibrinogen sealant comprising fibrinogen, thrombin, and CaCl₂ on at least one surface of said polymer film comprises placing a sufficient quantity of said fibrinogen sealant on at least one surface of said polymer film sufficient to provide between 0.5 mg and 8 mg of fibrinogen and between 20 IU and 1000 IU of thrombin per square centimeter of film.

14. The tissue adhesive patch according to any one of claims 1 -5 for use in the treatment of a leak of fluid into or out of a body part.

15. The tissue adhesive patch according to claim 14, **characterized in that** said treatment comprises applying a tissue adhesive patch according to any one of claims 1-5 to said body part, thereby causing said tissue adhesive patch to adhere to said body part, thereby sealing said body part.

## Patentansprüche

1. Gewebeheftpflaster auf Basis von Fibrinogen, umfassend:
einen Träger, hergestellt aus einer Folie, die aus einem biokompatiblen Polymer hergestellt ist; und
ein Fibrinogendichtmittel;
**dadurch gekennzeichnet, dass**
das biokompatible Polymer ein Polyethylenglykol-Polycaprolacton-DL-Lactid-Copolymer ist;
das Fibrinogendichtmittel in den biokompatiblen Polymerträger eingearbeitet ist; und das Gewebeheftpflaster keine Netz- oder gewebte Komponente beinhaltet.

2. Gewebeheftpflaster nach Anspruch 1, **dadurch gekennzeichnet, dass** das Fibrinogendichtmittel im Wesentlichen aus Fibrinogen, Thrombin und CaCl₂ besteht.

3. Gewebeheftpflaster nach Anspruch 1, **dadurch gekennzeichnet, dass** das Gewebeheftpflaster kein blutstillendes Mittel in Form eines freien Pulvers umfasst.

4. Gewebeheftpflaster nach Anspruch 1, **dadurch gekennzeichnet, dass** das Pflaster zwischen 0,5 mg und 8 mg Fibrinogen und zwischen 20 IE und 1000 IE Thrombin je Quadratzentimeter Folie umfasst.

5. Gewebeheftpflaster nach Anspruch 1, **dadurch gekennzeichnet, dass** das Fibrinogendichtmittel zusätzlich mindestens ein Additiv umfasst.

6. Verfahren zur Herstellung eines Gewebeheftpflasters auf Basis von Fibrinogen, **dadurch gekennzeichnet, dass** das Verfahren umfasst:
Gießen einer Polymerfolie aus einem biokompatiblen Polymer;
Erweichen der Polymerfolie;
Aufbringen eines Fibrinogendichtmittels auf mindestens eine Oberfläche der Polymerfolie; und
Pressen der Polymerfolie, bis mindestens ein Teil des Fibrinogendichtmittels in die Oberfläche der Polymerfolie eingearbeitet ist.

7. Verfahren nach Anspruch 6, **dadurch gekennzeichnet, dass** der Schritt des Aufbringens eines Fibrinogendichtmittels auf mindestens eine Oberfläche der Polymerfolie das Aufbringen eines Fibrinogendichtmittels, das Fibrinogen, Thrombin und CaCl₂ umfasst, auf mindestens eine Oberfläche der Polymerfolie umfasst.

8. Verfahren nach Anspruch 6, **dadurch gekennzeichnet, dass** das biokompatible Polymer undurchlässig ist.

9. Verfahren nach Anspruch 6, **dadurch gekennzeichnet, dass** das biokompatible Polymer aus der Gruppe ausgewählt ist, bestehend aus Polyethylenglykol-Polycaprolacton-Copolymeren; Polyethylenglykol-DL-Lactid-Copolymeren; und Polyethylenglykol-Polycaprolacton-DL-Lactid-Copolymeren.

10. Verfahren nach Anspruch 6, **dadurch gekennzeichnet, dass** der Schritt des Gießens einer Polymerfolie umfasst:
Herstellen einer Lösung eines trockenen Polymers in einem organischen Lösungsmittel; und
Verdampfen des organischen Lösungsmittels.

11. Verfahren nach Anspruch 6, **dadurch gekennzeichnet, dass** der Schritt des Gießens einer Polymerfolie das Gießen der Polymerfolie auf eine glatte flache Oberfläche umfasst, die aus einem Material hergestellt ist, das aus der aus Glas, Silikon und Polytetrafluorethylen bestehenden Gruppe ausgewählt ist.

12. Verfahren nach Anspruch 11, **dadurch gekennzeichnet, dass** das Verfahren das Platzieren der Polymerfolie in einer Tiefkühlvorrichtung nach dem Schritt des Pressens des Polymers und Entfernen der Polymerfolie von der glatten flachen Oberfläche nach dem Schritt des Platzierens der Polymerfolie in einer Tiefkühlvorrichtung umfasst.

13. Verfahren nach Anspruch 6, **dadurch gekennzeichnet, dass** der Schritt des Aufbringens eines Fibrinogendichtmittels, umfassend Fibrinogen, Thrombin und CaCl₂, auf mindestens eine Oberfläche der Polymerfolie das Aufbringen einer ausreichenden Menge des Fibrinogendichtmittels auf mindestens eine Oberfläche der Polymerfolie umfasst, die ausreicht, um zwischen 0,5 mg und 8 mg Fibrinogen und zwischen 20 IE und 1000 IE Thrombin je Quadratzentimeter Folie vorzusehen.

14. Gewebeheftpflaster nach einem der Ansprüche 1-5 zur Anwendung bei der Behandlung einer Undichtigkeit von Fluid in ein oder aus einem Körperteil.

15. Gewebeheftpflaster nach Anspruch 14, **dadurch gekennzeichnet, dass** die Behandlung das Anbringen eines Gewebeheftpflasters nach einem der Ansprüche 1-5 an dem Körperteil umfasst, wodurch veranlasst wird, dass das Gewebeheftpflaster an dem Körperteil haftet, wodurch der Körperteil abgedichtet wird.

## Revendications

1. Emplâtre adhésive tissulaire à base de fibrinogène, comprenant :
un support constitué d'un film réalisé à partir d'un polymère biocompatible ; et
un agent d'étanchéité à base de fibrinogène ;
**caractérisée en ce que** :
ledit polymère biocompatible est un copolymère de polyéthylène glycol-polycaprolactone-DL-lactide ;
ledit agent d'étanchéité à base de fibrinogène est incorporé dans ledit support constitué d'un polymère biocompatible ; et
ladite emplâtre adhésive tissulaire n'englobe aucun composant à mailles ou tissé.

2. Emplâtre adhésive tissulaire selon la revendication 1, **caractérisée en ce que** ledit agent d'étanchéité à base de fibrinogène est constitué essentiellement de fibrinogène, de thrombine et de CaCl₂.

3. Emplâtre adhésive tissulaire selon la revendication 1, **caractérisée en ce que** ladite emplâtre adhésive tissulaire ne comprend aucun agent hémostatique sous la forme d'une poudre libre.

4. Emplâtre adhésive tissulaire selon la revendication 1, **caractérisée en ce que** ladite emplâtre comprend entre 0,5 mg et 8 mg de fibrinogène et entre 20 UI et 1.000 UI de thrombine par centimètre carré de film.

5. Emplâtre adhésive tissulaire selon la revendication 1, **caractérisée en ce que** ledit agent d'étanchéité à base de fibrinogène comprend en outre au moins un additif.

6. Procédé pour la production d'une emplâtre adhésive tissulaire à base de fibrinogène, **caractérisé en ce que** ledit procédé comprend le fait de :
obtenir par moulage un film polymère à partir d'un polymère biocompatible ;
ramollir ledit film polymère ;
placer un agent d'étanchéité à base de fibrinogène sur au moins une surface dudit film polymère ; et
comprimer ledit film polymère jusqu'à incorporation d'au moins une portion dudit agent d'étanchéité à base de fibrinogène dans la surface dudit film polymère.

7. Procédé selon la revendication 6, **caractérisé en ce que** ladite étape consistant à placer un agent d'étanchéité à base de fibrinogène sur au moins une surface dudit film polymère comprend le fait de placer un agent d'étanchéité à base de fibrinogène comprenant du fibrinogène, de la thrombine et du CaCl₂ sur au moins une surface dudit film polymère.

8. Procédé selon la revendication 6, **caractérisé en ce que** ledit polymère biocompatible est non perméable.

9. Procédé selon la revendication 6, **caractérisé en ce que** ledit polymère biocompatible est choisi parmi le groupe constitué par des copolymères de polyéthylène glycol-polycaprolactone, des copolymères de polyéthylène glycol-DL-lactide et des copolymères de polyéthylène glycol-polycaprolactone-DL-lactide.

10. Procédé selon la revendication 6, **caractérisé en ce que** ladite étape consistant à l'obtention d'un film polymère par moulage comprend le fait de :
préparer une solution d'un polymère sec dans un solvant organique ; et
éliminer ledit solvant organique par évaporation.

11. Procédé selon la revendication 6, **caractérisé en ce que** ladite étape consistant à l'obtention d'un film polymère par moulage comprend le fait de couler ledit film polymère sur une surface plate lisse réalisée à partir d'un matériau choisi parmi le groupe constitué par du verre, du silicone et du polytétrafluoréthylène.

12. Procédé selon la revendication 11, **caractérisé en ce que** ledit procédé comprend le fait de placer ledit film polymère dans un congélateur suite à ladite étape consistant à comprimer le polymère et le fait de retirer ledit film polymère de ladite surface plate lisse après ladite étape de placement dudit film polymère dans un congélateur.

13. Procédé selon la revendication 6, **caractérisé en ce que** ladite étape consistant à placer un agent d'étanchéité à base de fibrinogène comprenant du fibrinogène, de la thrombine et du CaCl₂ sur au moins une surface dudit film polymère comprend le fait de placer une quantité suffisante dudit agent d'étanchéité à base de fibrinogène sur au moins une surface dudit film polymère, suffisante pour obtenir entre 0,5 mg et 8 mg de fibrinogène et entre 20 UI et 1.000 UI de thrombine par centimètre carré de film.

14. Emplâtre adhésive tissulaire selon l'une quelconque des revendications 1 à 5, pour son utilisation dans le traitement d'une fuite d'un fluide depuis l'extérieur jusque dans une partie du corps ou depuis l'intérieur de celle-ci vers l'extérieur.

15. Emplâtre adhésive tissulaire selon la revendication 14, **caractérisée en ce que** ledit traitement comprend le fait d'appliquer une emplâtre adhésive tissulaire selon l'une des revendications 1 à 5 sur ladite partie du corps, tant et si bien que ladite emplâtre adhésive tissulaire adhère à la partie du corps pour ainsi fermer de manière hermétique ladite partie du corps.
